# EUROPEAN PATENT SPECIFICATION

(11) **EP 1 236 715 B1**
(45) Date of publication and mention of the grant of the patent: **15.10.2008**
(21) Application number: 02009545.1
(22) Date of filing: 24.06.1999
(51) Int. Cl.: C07C 323/59, C07C 319/14, C07C 227/16

(54) **Process for S-Aryl cysteine**
Verfahren zur Herstellung von S-Aryl-Cystein
Procédé pour la préparation de la S-aryl cystéine

(30) Priority: 29.06.1998 US 91102 P; 11.08.1998 US 96142 P
(43) Date of publication of application: 04.09.2002
(62) Divisional of application: 99111959.5
(73) Proprietor: F. Hoffmann-La Roche AG, 4070 Basel (CH)
(72) Inventor: Brown, Jack D., Westminster, Colorado 80020 (US); Dauer, Richard, Longmont, Colorado 80503 (US); Harrington, Peter John, Louisville, Colorado 80027 (US); Johnston, Dave A., Louisville, Colorado 80027 (US); Khatri, Hiralal N., Louisville, Colorado 80027 (US); Rowe, Gary K., Arvada, Colorado 80004 (US); Topping, Robert J., Longmont, Colorado 80503 (US)
(74) Representative: Rauber, Beat

(56) References cited:
- EP-A- 0 897 911
- WO-A-98/45271
- KNIGHT D W ET AL: "TOTAL SYNTHESIS OF (-)-SLAFRAMINE FROM (2R,3S)-HYDROXYPROLINE" JOURNAL OF THE CHEMICAL SOCIETY, PERKIN TRANSACTIONS 1, CHEMICAL SOCIETY. LETCHWORTH, GB, 1997, pages 2179-2187, XP001040653 ISSN: 0300-922X
- SASAKI N A ET AL: "A novel approach to the synthesis of optically pure non-protein alpha-amino acids in both L and D configurations from L-serine" TETRAHEDRON LETTERS, ELSEVIER SCIENCE PUBLISHERS, AMSTERDAM, NL, vol. 28, no. 48, 1987, pages 6069-6071, XP002157105 ISSN: 0040-4039
- PORTEVIN B ET AL: "DUAL INHIBITION OF HUMAN LEUKOCYTE ELASTASE AND LIPID PEROXIDATION:IN VITRO AND IN VIVO ACTIVITIES OF AZABICYCLOÄ2.2.2ÜOCTANE AND PERHYDROINDOLE DERIVATIVES" JOURNAL OF MEDICINAL CHEMISTRY, AMERICAN CHEMICAL SOCIETY. WASHINGTON, US, vol. 40, 1997, pages 1906-1918, XP002935499 ISSN: 0022-2623

## Description

S-aryl cysteines are useful intermediates in the synthesis of a variety of pharmaceutically active compounds. Chiral S-aryl-L-cysteines have been used successfully to target the human immunodeficient virus (HIV) and are used in the treatment of AIDS (Kaldor et al., J. Med. Chem. 1997, 40 (24), 3979-3985).

Representative potent and tight binding inhibitors of the human immunodeficient virus protease which contain an arylthio group are shown in Figure 1.

Many currently available synthetic methods for S-aryl cysteines involve the preparation of racemic mixtures. There are, however, a number of disadvantages associated with racemic mixtures of such compound. A racemic mixture of an S-aryl cysteine results in production of racemic drugs. It is well known that certain physiological properties of chiral drugs are dependent upon stereochemistry of the drug and the undesired side-effects are often attributed to the presence of the undesired stereoisomer of the chiral drug. Accordingly, a high enantioselective synthesis of a chiral drug will result in a drug having a desired therapeutic activity with a reduced amount of undesired side-effects. Of course, the synthesis of a chiral drug can include a step of separating a racemic mixture; however, this is often time consuming and costly. In addition, racemic synthesis requires discarding one half of the compound unless the undesired isomer can be converted to a desired isomer. Moreover, not all racemic compounds can be resolved to provide a satisfactory yield of a desired enantiomer.

Current methods for enantioselective synthesis of S-aryl cysteines involve enzymatic methods (see, e.g., USP 5.756.319 or European Patent Application No. 754,759, which are assigned to Mitsui Toatsu Chemicals, Inc.), applicable, however, to the preparation of only a limited number of S-aryl cysteines.

Most of the current chemical synthetic methods for enantioselective preparation of S-aryl cysteines result in a racemic mixture, use elaborate reagents dramatically increasing the overall cost, or result in too low levels of enantioselectivity to be useful in a pharmaceutical process.

Recently, D.W. Knight and A.W. Sibley (J. Chem. Soc., Perkin Trans. 1, 1997, 2179-2187) reported that reaction of methyl (S)-2-benzyloxycarbonylamino-3-methylsulfonyloxy-propanoate with freshly prepared sodium thiophenylate in DMF at about 0°C afforded the desired N-carbobenzyloxy-S-phenyl-L-cysteine methyl ester (or methyl (R)-2-benzyloxycarbonylamino-3-phenylthiopropanoate) in 98% yield providing a reported optical rotation of [α]²⁰_{D} - 17.2 (c, 1.8; MeOH). No enantiomeric ratio of the product was reported. Furthermore, the use of sodium phenolate, prepared from sodium hydride, thiophenol and DMF is not amenable to large scale manufacture.

Therefore, there is a need for an efficient, concise and enantioselective method suitable for the large scale manufacture of S-aryl cysteines using relatively inexpensive reagents.

### Definitions

"Alkyl" comprises straight or branched chain groups having 1 to about 10 carbon atoms. Alkyl groups optionally can be substituted with one or more substituents, such as halogen, aryl, hydroxy, alkoxy, carboxy, oxo and cycloalkyl. There may be optionally inserted along the alkyl group one or more oxygen, sulfur or substituted or unsubstituted nitrogen atoms. Exemplary alkyl groups include methyl, ethyl, *i*-propyl, *n*-butyl, *t*-butyl, *n*-pentyl, heptyl, benzyl, and octyl.

"Aryl" means an aromatic group which has at least one ring which has a conjugated pi electron system and includes, without limitation, carbocyclic aryl, heterocyclic aryl, biaryl groups and heterocyclic biaryl, all of which can be optionally substituted.

"Heterocyclic aryl groups" refer to groups having at least one heterocyclic aromatic ring containing from 1 to 3 heteroatoms in the ring with the remainder being carbon atoms. Suitable heteroatoms include, without limitation, oxygen, sulfur, and nitrogen. Exemplary heterocyclic aryl groups include furanyl, thienyl, pyridyl, pyrrolyl, N-alkyl pyrrolo, pyrimidyl, pyrazinyl, imidazolyl, benzofuranyl, quinolinyl, and indolyl.

Preferably, the aryl group is substituted or unsubstituted aryl selected from the group consisting of phenyl, naphthyl, pyridyl, furyl, thiophenyl, pyrazyl and pyrrolyl. More preferably, the aryl group is substituted or unsubstituted aryl group selected from the group consisting of phenyl, naphthyl and pyridyl, still more preferably the aryl group is selected from the group consisting of substituted and unsubstituted phenyl, and most preferably the aryl group is phenyl.

The term "S-aryl" refers to a substituent wherein an aromatic group is bonded to a sulfur atom. "S-aryl" group may also be referred to as an "aryl thioether" group. The term "S-arylation" refers to the process of substituting a compound or groups with an S-aryl group.

The term "metal" comprises alkali metals, alkaline-earth metals, transition metals, noble metals, platinum metals, rare metals, rare-earth metals, actinide metals, light metals, and heavy metals. Examples of such metals are aluminum, iron, copper, cobalt, potassium, sodium, tin and zinc.

The term "catalyst" refers to any substance of which a fractional percentage notably affects the rate of a chemical reaction without itself being consumed or undergoing a chemical change, as defined in "Hawley's Condensed Chemical Dictionary", 11th ed., revised by N. Irving Sax and Richard J. Lewis, Sr., Van Nostrand Reinhold Company, New York, p. 748 (1987).

The term "stoichiometric" refers to the use or addition of an equivalent mole ratio or amount of a reagent relative to a selected substrate, molecule, or compound in a reaction.

The term "chiral" has the usual meaning known to a person skilled in the art.

The term "enantiomeric excess" refers to a difference between the amount of one enantiomer and the amount of the other enantiomer that is present in the product mixture. Thus for example, enantiomeric excess of 96% refers to a product mixture having 98% of one enantiomer and 2% of the other enantiomer.

The following abbreviations and terms are used herein:
"CBZ" means benzyloxycarbonyl or carbobenzyloxy.
"DMF" means dimethylformamide.
"Tosylate" or "Tos" means p-toluenesulfonate ester.
"Mesylate" or "Ms" means methanesulfonate ester.
"TBPB" means tetrabutylphosphonium bromide.
"TBAB" means tetrabutylammonium bromide.
"TBAC" means tetrabutylammonium chloride.
"PTC" means phase transfer catalysis or catalyst.
"Aliquat 336®" is tricaprylylmethylammonium chloride (TCMC).

The present invention is directed to a method for preparing S-aryl cysteines (which are useful intermediates in a variety of pharmaceutically active compounds) in enantiomeric excess of greater than 96%, more preferably greater than 98%, and most preferably greater than 99.5%. Unless the context requires otherwise, reference to any compound is to be considered as a reference to an individual enantiomer of the compound, and to racemic or non-racemic mixtures thereof.

### BRIEF DESCRIPTION OF FIGURES

Figure 1 shows representative compounds of HIV protease inhibitors which contain a moiety derived from S-aryl cysteine.
Figure 2 illustrates the present invention for preparing an S-aryl cysteine compound which utilizes serine.

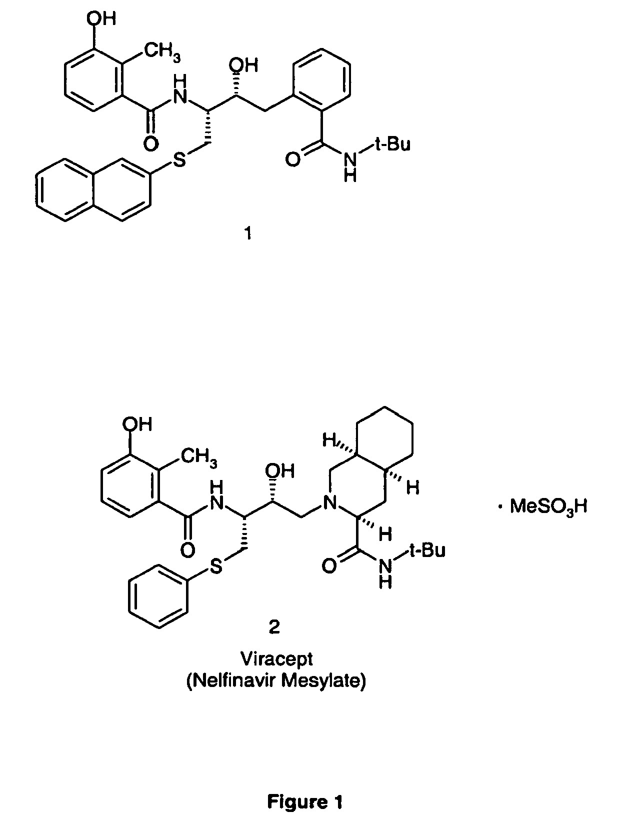

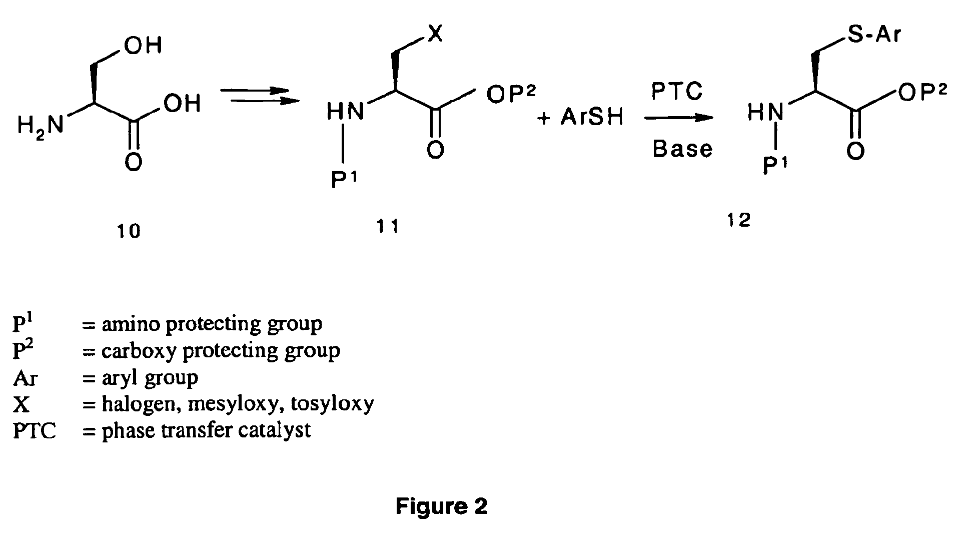

The present invention comprises a method for preparing an S-aryl cysteine in enantiomeric excess of greater than 96% comprising contacting a compound of the formula 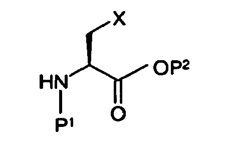 wherein X is a halogen atom, a mesyloxy or tosyloxy group;
P¹ is an amino protecting group and
P² is a carboxy protecting group,
with an aryl thiol in the presence of a base selected from sodium bicarbonate or potassium carbonate, a phase transfer catalyst selected from TBAB (tetra butyl ammonium bromide) or TBPB (tetrabutyl phosphonium bromide) in toluene as solvent. It will be appreciated that the aryl thiol can be contacted with the base prior to adding any serine derivative with a leaving group, or the base can be added to a mixture of the aryl thiol and the serine derivative, or the aryl thiol can be added to the mixture of the base and the serine derivative. In a particular aspect the serine derivative may be prepared from serine by protection of the amino group and the carboxy group followed by conversion of the hydroxy group to a leaving group.

The temperature of the reaction can affect the enantiomeric excess of the product. To minimize loss of stereochemical configuration of the product and/or the starting material, the temperature of the reaction between the serine derivative and the aryl thiol is maintained from about -5°C to about 35°C, preferably from about 15°C to about 30°C. Preferably the reaction time is from about 1 h to about 48 hs, more preferably from about 10 hs to about 30 hs, and most preferably from about 20 hs to about 25 hs.

The above methods of the present invention can include protecting the amino group of the amino acid (cystine, cysteine or serine). Any of the known amino protecting groups can be used. Examples of some protecting groups are described in "Solid Phase Peptide Synthesis" by G. Barany and R. B. Merrifield in Peptides, Vol. 2, edited by E. Gross and J. Meienhoffer, Academic Press, New York, N.Y., pp. 100-118 (1980), and "Protective Groups in Organic Synthesis" by Green, T., John Wiley & Sons, Inc., New York, NY., 1981, pp. 218-287. Exemplary N-amino protecting groups include acetyl, formyl, benzoyl, substituted benzoyls, FMOC, Bspoc, Bsmoc, t-butyloxycarbonyl (BOC), t-amyloxycarbonyl (Mcb), 2-(p-biphenylyl)-propyl-2-oxycarbonyl (Bpoc), benzyloxycarbonyl (or carbobenzyloxy, CBZ), phthaloyl, piperidino-oxycarbonyl, trifluoroacetyl and the like. Other N-amino protecting groups include optionally protected α-amino acids which are linked with the carboxyl moiety of the α-amino acids. Preferably the amino protecting group is metyl carbamate or CBZ. Amino protecting group can be removed under various conditions, including mild acidic or basic conditions. The preferred protecting groups are those which can be cleaved by an acid or a base, or reductive conditions. For example, the amino group can be protected by contacting the amino acid with benzyl chloroformate in the presence of a base. Any base that can neutralize the acidic proton that is formed by the reaction of benzyl chloroformate and the amino group can be used. Exemplary bases useful in protection of the amino group include carbonates such as sodium carbonate, potassium carbonate and lithium carbonate; bicarbonates such as sodium bicarbonate, potassium bicarbonate and lithium bicarbonate; hydroxides such as sodium hydroxide, lithium hydroxide, calcium hydroxide, magnesium hydroxide and potassium hydroxide; and sterically hindered amines such as triethyl amine and diisopropyl ethyl amine. Preferably, the base is selected from the group consisting of carbonates, bicarbonates and hydroxides, more preferably the base is selected from the group consisting of carbonates and bicarbonates, and most preferably the base is a bicarbonate. In one particular embodiment of the present invention, the amino group is protected by contacting the amino acid with benzyl chloroformate in the presence of sodium bicarbonate to provide N-benzyloxycarbonyl protected amino acid.

For the protection of the carboxy group of the amino acid any of the known carboxy protecting groups can be used. Protection of the carboxy moiety of amino acids are described in "The Peptides," E. Gross and J. Meienhofer, Eds., Vol.3, Academic Press, NY (1981), pp. 101-135, and "Protective Groups in Organic Synthesis" by Green, T., John Wiley & Sons, Inc., New York, NY., 1981, pp. 152-192. Exemplary carboxy protecting groups include esters such as alkyl esters including methyl, ethyl, tert-butyl, methoxymethyl, 2,2,2-trichloroethyl and 2-haloethyl; benzyl esters such as triphenylmethyl, diphenylmethyl, *p*-bromobenzyl, *o*-nitrobenzyl and the like; silyl esters such as trimethylsilyl, triethylsilyl, *t*-butyldimethylsilyl and the like; amides and hydrazides. Other carboxy protecting groups can include optionally protected α-amino acids which are linked with the amino moiety of the α-amino acids. Preferably the carboxylic acid protecting group is an ester, more preferably the carboxylic acid protecting group is an alkyl ester, and most preferably the carboxylic acid protecting group is selected from the group consisting of methyl ester and ethyl ester. In a particular embodiment of the present invention, the carboxylic acid of the amino acid is protected as methyl ester by contacting the amino acid with thionyl chloride in the presence of methanol. Alternatively, the carboxylic acid is protected as methyl ester by contacting the amino acid with gaseous hydrochloric acid in the presence of methanol.

The above method can also include protecting both the amino group and the carboxy group of the amino acid. It will be appreciated that the amino and carboxy groups in the amino acid can be protected in any sequence.

The S-aryl cysteine reaction product of the present invention which is recovered from the reaction mixture can be further purified by distillation or crystallization. For instance, a reaction product obtained in liquid form, is dissolved in toluene and crystallized from a relatively non-polar recrystallization solvent to afford a product of higher purity. Preferably, the non-polar recrystallization solvent is selected from the group consisting of hexane, ethyl acetate, toluene, xylene, benzene, pentane, ethers and mixtures thereof. The S-aryl cysteine can be recovered as a salt. For example, adding an acid such as hydrochloric acid; or an organic acid including tartaric acid, acetic acid, and/or citric acid to S-aryl cysteine can result in the formation of the corresponding S-aryl cysteine salt which can be easily isolated. Alternatively, the free carboxy group can react with a base to generate a carboxylate salt which can form a solid. In yet another alternative, the presence of both free amino and carboxy groups results in the formation of a zwitter ion which can precipitate as a solid.

The present invention is further illustrated by the following examples

### EXAMPLES

### Phase Transfer Catalyzed Nucleophilic Displacement Reactions

Phase-transfer catalyzed nucleophilic displacement of N-protected serine ester mesylate (Ms) or tosylate (Tos) using aryl thiols to prepare the corresponding N-protected S-aryl-L-cysteine derivatives is provided in the following representative experimental descriptions. Tables 1-3 provide results from various alkylation reactions employing different reactions conditions, including variations in the type of phase-transfer catalyst, reaction stoichiometry, base, and substrates.

**Table 1.**

| **Phase Transfer Alkylation with Thiophenol** | | | |
|---|---|---|---|
| | 1 | 2 | 3 |
| **Substrate** | mesylate | mesylate | mesylate |
| **PhSH (equiv)** | 1.0 | 1.0 | 1.0 |
| **PTC** | TBAB | TBAB | TBAB |
| **PTC (mol%)** | 5.0 | 10 | 10 |
| **Base** | K₂CO₃ | NaHCO₃ | K₂CO₃ |
| **Base (equiv)** | 1.20 | 1.10 | 2.00 |
| **Solvent (ml)** | toluene (10) | toluene (10) | toluene(10) |
| **Temp (°C)** | 25 | 25 | 25 |
| **Time (h)** | 22 | 23 | 22 |
| **Crude Yield (g)** | --- | --- | --- |
| **Chrom Yield (g)** | 0.255 | 0.411 | 0.301 |
| **Chrom Yield (%)** | 24.5 | 39.4 | 28.9 |
| **R:S** | 99.5:0.5 | 98.4:1.6 | 99.1:0.9 |
| **ee (%)** | 99.0 | 96.8 | 98.2 |

**Table 2.**

| **Phase Transfer Alkylation with Thiophenol: Tosylate as Substrate** | | |
|---|---|---|
| | 4 | 5 |
| **Substrate** | tosylate | tosylate |
| **PhSH (equiv)** | 1.0 | 1.0 |
| **PTC** | TBAB | TBPB |
| **PTC (mol%)** | 5.0 | 5.0 |
| **Base** | K₂CO₃ | K₂CO₃ |
| **Base (equiv)** | 1.50 | 1.50 |
| **Solvent (ml)** | toluene (10) | toluene (10) |
| **Temp (°C)** | 25 | 25 |
| **Time (h)** | 17 | 24 |
| **Crude Yield (g)** | 0.959 | 0.986 |
| **Chrom Yield (g)** | 0.877 | 0.858 |
| **Chrom Yield (%)** | 84.2 | 82.3 |
| **R:S** | 99.2:0.8 | 99.8:0.2 |
| **ee (%)** | 98.4 | 99.6 |

**Table 3.**

| **Phase Transfer Alkylation with Thiophenol** | |
|---|---|
| | 6 |
| **Substrate** | mesylate |
| **PhSH (equiv)** | 1.0 |
| **PTC** | TBPB |
| **PTC (mol%)** | 5.0 |
| **Base** | K₂CO₃ |
| **Base (equiv)** | 1.50 |
| **Solvent (m)** | toluene (10) |
| **Temp (°C)** | 25 |
| **Time (h)** | 20.5 |
| **Crude Yield (g)** | 0.948 |
| **Chrom Yield (g)** | 0.409 |
| **Chrom Yield (%)** | 39.2 |
| **R:S** | 98.2:1.8 |
| **ee (%)** | 96.4 |

### EXAMPLE 7

### Preparation of N-CBZ S-phenyl-L-cysteine methyl ester by the displacement of N-carbobenzyloxy-O-p-toluenesulfonyl-L-serine methyl ester.

A mixture of 1.231 g (3.02 mmol) of N-carbobenzyloxy-O-p-toluenesulfonyl-L-serine methyl ester, anhydrous potassium carbonate powder (0.626 g, 4.53 mmol, 1.5 equiv.), tetrabutylphosphonium bromide (TBPB, 51 mg, 0.151 mmol, 5 mole %), thiophenol (0.31 ml, 3.02 mmol), and toluene was stirred at 25°C for 24 hours.

Water (20 ml) was added and the phases were separated. The organic phase was washed with water (20 ml), dried over magnesium sulfate, and filtered. Concentration on a rotoevaporator at 30-35°C and 4 kPa (30 mmHg) afforded an oil. Drying of the oil under vacuum at 25°C for 18 hours (ca. 66.6 Pa (0.5 mmHg)) afforded a colorless solid (0.986 g, 94.6% yield).

### EXAMPLE 8

### Preparation of N-CBZ S-phenyl-L-cysteine methyl ester by the displacement of N-carbobenzyloxy-O-methanesulfonyl-L-serine methyl ester.

Thiophenol (0.31 ml, 0.333 g, 3.02 mmol) was added *via* syringe to a suspension of N-carbobenzyloxy-O-methanesulfonyl-L-serine methyl ester (1.00 g, 3.02 mmol), powdered anhydrous K₂CO₃, tetrabutylammonium bromide (TBAB, 49 mg, 0.151 mmol, 5 mole%), and toluene (20 ml). The suspension was stirred at 25°C for 22 hours. Water (20 ml) was added and the phases were separated. The organic phase was washed with 20 ml water, 10 ml of ethyl acetate was added, dried over magnesium sulfate, and filtered. Concentration on a rotoevaporator in vacuo at 35°C and 4.66 kPa (35 mmHg) afforded a wet colorless solid.

The solid was dissolved in methylene chloride and separated on a 4 mm silica gel chromatotron plate. The product was separated using hexane (250 ml), 10% ethyl acetate in hexane (800 ml), ethyl acetate (200 ml), and methanol (200 ml). The combined fractions were concentrated on a rotoevaporator at 10 kPa (75 mmHg) and 30-35°C. The residual oil was triturated with hexane and the solids were dried under vacuum (66.6 Pa (0.5 mmHg)) for 7.5 hours at 25°C to afford 0.255 g of a colorless solid (24.5% chromatographed yield, 98.2% R : 0.50% S by assay).

### EXAMPLE 9

### Preparation of N-CBZ S-phenyl-L-cysteine methyl ester from N-carbobenzyloxy-O-p-toluenesulfonyl-L-serine methyl ester.

A mixture of 12.31 g (30.2 mmol) of N-carbobenzyloxy-O-p-toluenesulfonyl-L-serine methyl ester 6.26 g (45.3 mmol, 1.5 equivalents) of anhydrous powdered potassium carbonate, 512 mg (1.51 mmol, 5.0 mol%) tetrabutylphosphonium bromide, 100 ml toluene, and 3.1 ml (3.33 g, 30.2 mmol) thiophenol was stirred at 25°C for 31 hours.

Reaction progress was followed by liquid chromatography (LC): After 1 h, 85% tosylate remaining, after 5 hs 55%, after 25 hs 4.6%, after 30 hs tosylate not detected any more. Water (40 ml) was added and the layers separated. The organic layer was washed with 20 ml of water then distilled at atmospheric pressure to reduce the volume of toluene (bath 125°C, under dry N₂). The weight of solution remaining after the distillation was 28.75 g.

The solution after cooling contained a small amount of solid residue (salts from residual water in the toluene after the phase split). These solids were removed by gravity filtration. The mother liquor was slowly diluted with 80 ml of hexane. The precipitate was suction filtered, washed on the funnel with 20 ml hexane, then dried under vacuum at 25°C for 22 h to afford 8.309 g of colorless solid, mp. 64.7-65.2°C; yield 79.9%. LC assay for optical purity: 0.1% S.

### EXAMPLE 10

### Preparation of N-CBZ S-phenyl-L-cysteine methyl ester from N-carbobenzyloxy-O-p-toluenesulfonyl-L-serine methyl ester.

A mixture of 30.00 g (73.63 mmol) of N-carbobenzyloxy-O-p-toluenesulfonyl-L-serine methyl ester, 15.27 g (110 mmol, 1.5 equivalents) of anhydrous powdered potassium carbonate 1.249 g (3.68 mmol, 5.0 mol%) of tetrabutylphosphonium bromide, 150 ml toluene, and 7.6 ml (8.11 g, 73.6 mmol) thiophenol was stirred at 25°C for 8 days.

Water (75 ml) was added and the layers separated. The organic layer was washed with 75 ml water and then distilled at reduced pressure to reduce the volume of toluene (heating bath at 125°C, under dry N₂). The weight of solution remaining after the distillation was 41.6 g.

The residual oil was diluted with 100 ml of hexane. The precipitate was suction filtered, washed on the funnel with 75 ml hexane, then dried under vacuum at 25°C for 19 hours to afford 22.75 g of colorless solid. yield 89.5%. LC assay for optical purity: 0.5% S. LC assay for chemical purity: 97.9%.

## Claims

1. A method for preparing an S-aryl cysteine in enantiomeric excess of greater than 96% comprising contacting a compound of the formula 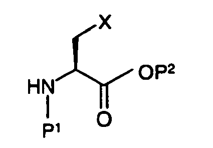 wherein X is a halogen atom, a mesyloxy or tosyloxy group;
P¹ is an amino protecting group and
P² is a carboxy protecting group,
with an aryl thiol in the presence of a base selected from sodium bicarbonate or potassium carbonate, a phase transfer catalyst, selected from the group consisting of TBAB and TBPB in toluene as solvent, wherein "Aryl" means an aromatic group which has at least one ring which has a conjugated pi electron system and includes, without limitation, carbocyclic aryl, heterocyclic aryl, biaryl groups and heterocyclic biaryl, all of which can be optionally substituted,
"Heterocyclic aryl groups" refer to groups having at least one heterocyclic aromatic ring containing from 1 to 3 heteroatoms in the ring with the remainder being carbon atoms, wherein suitable heteroatoms include, without limitation, oxygen, sulfur, and nitrogen.

2. The method of Claim 1, wherein the amino protecting group P¹ is selected from the group consisting of methyl carbamate and carbobenzyloxy.

3. The method of of Claim 1, wherein the carboxy protecting group P² is the methyl ester group.

## Patentansprüche

1. Verfahren zur Herstellung eines S-Aryl-Cysteins in enantiomerem Überschuß von mehr als 96 % umfassend, dass eine Verbindung der Formel 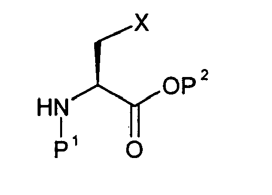 worin X ein Halogen-Atom, eine Mesyloxy- oder eine Tosyloxy-Gruppe ist;
P¹ eine Aminoschutzgruppe ist und
P² eine Carboxylschutzgruppe ist,
mit einem Arylthiol in Kontakt gebracht wird, in Anwesenheit einer Base ausgewählt aus Natriumbikarbonat oder Kaliumkarbonat, einem Phasen-Transfer-Katalysator ausgewählt aus der Gruppe bestehend aus TBAB und TBPB in Toluol als Lösungsmittel,
worin
"Aryl" eine aromatische Gruppe bedeutet, die mindestens einen Ring aufweist, der ein konjugiertes pi-Elektronensystem hat und ohne Beschränkung carbocyclische Aryl-, heterocyclische Aryl-, Biarylgruppen und heterocyclische Biarylgruppen einschließt, die alle gegebenenfalls substituiert sein können,
"heterocyclische Aryl-Gruppen" sich auf Gruppen beziehen mit mindestens einem heterocyclischen aromatischen Ring, der 1 bis 3 Heteroatome im Ring aufweist, wobei der Rest Kohlenstoffatome sind, wobei geeignete Heteroatome ohne Beschränkung Sauerstoff, Schwefel und Stickstoff einschließen.

2. Verfahren nach Anspruch 1, worin die Aminoschutzgruppe P¹ ausgewählt ist aus der Gruppe bestehend aus Methylcarbamat und Carbobenzyloxy.

3. Verfahren nach Anspruch 1, worin die Carboxylschutzgruppe P² die Methylestergruppe ist.

## Revendications

1. Procédé pour préparer une cystéine S-aryle avec un excédent énantiomérique supérieur à 96 %, comprenant la mise en contact d'un composé de la formule 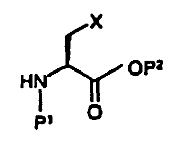 où X est un atome halogène, un groupe mésyloxy ou tosyloxy ;
P¹ est un groupe protecteur aminé et
P² est un groupe protecteur carboxy,
avec un aryl thiol en présence d'une base choisie parmi le bicarbonate de sodium ou le carbonate de potassium, un catalyseur de transfert de phase, choisi dans le groupe consistant en TBAB et TBPB dans le toluène en tant que solvant,
dans lequel "aryle" signifie un groupe aromatique qui possède au moins un cycle qui présente un système d'électrons pi conjugués et comprend, sans limitation, aryle carbocyclique, aryle hétérocyclique, des groupes biaryle et biaryle hétérocyclique, dont tous peuvent être facultativement substitués,
les "groupes aryle hétérocycliques" se rapportent aux groupes ayant au moins un cycle aromatique hétérocyclique contenant de 1 à 3 hétéroatomes dans le cycle, le reste étant constitué d'atomes de carbone, dans lequel les hétéroatomes appropriés comprennent, sans limitation, l'oxygène, le soufre et l'azote.

2. Procédé selon la revendication 1, dans lequel le groupe protecteur aminé P¹ est choisi dans le groupe consistant en méthyl carbamate et carbobenzyloxy.

3. Procédé selon la revendication 1, dans lequel le groupe protecteur carboxy P² est le groupe méthyl ester.
